# EUROPEAN PATENT APPLICATION

(11) **EP 1 953 135 A1**
(43) Date of publication of application: **06.08.2008**
(21) Application number: 07101716.4
(22) Date of filing: 05.02.2007
(51) Int. Cl.: C07C 67/08, C08K 5/12, C07C 69/75

(54) **Method of preparing cyclohexanepolycarobxylic acid ester without phthalatic and plasticizer prepared by the same**

(71) Applicant: NAN YA PLASTICS CORPORATION, Taipei (TW)
(72) Inventor: Shieh, Sung-Yueh Nan Ya Plastics Corporation, Taipei (TW); Fung, Dein-Run Nan Ya Plastics Corporation, Taipei (TW); Hsu, Han-Ching Nan Ya Plastics Corporation, Taipei (TW); Liu, Yang-Tu Nan Ya Plastics Corporation, Taipei (TW); Lin, Hsun-Min Nan Ya Plastics Corporation, Taipei (TW)
(74) Representative: Beck, Michael Rudolf

(57) **Abstract**

A method for preparing a kind of plasticizer containing cyclohexane poly carboxylic acid ester, particularly without phthalate, and with the advantage of effective shortening of reaction time, which method comprising having cyclohexane polycarboxylic acid or its derivatives, mono-alcohol and metal catalyst of tin and titanium or inorganic acid added together at the same time into a reactor to undergo a single stage of esterification at high temperature of 200~250°C, and a reaction mixture of cyclohexane polycarboxylic acid ester was obtained by the process which is applicably used as a plasticizer without phthalate and no hazard to the living beings and environment.

## Description

### BACKGROUND OF THE PRESENT INVENTION

### 1. Field of the Present Invention

The invention relates to a method of preparing a cyclohexanepolycarboxylic acid ester, particularly the cyclohexanepolycarboxylic acid ester without any phthalatic acid applicably used as a plasticizer to supersede the traditional phthalate group plasticizer in application.

### 2. Description of Prior Act

Plasticizers are widely used in many ways in plastics, coating compositions, sealing compositions and additive. They interact physically with thermoplastic high polymers without reacting chemically, preferably by means of their solvent and swelling capability. This forms a homogeneous system whose thermoplastic range has been shifted to lower temperatures compared to the original polymer, with the result that, for example, the ability to change shape and the elasticity are increased and the hardness is reduced.

To open up very widely fields of application for plasticizers, they have to fulfill a number of requirements. In the ideal case, they should be odorless, colorless, light-resistant, cold-resistant and heat-resistant. In addition, it is expected that they are resistant to water, do not bum readily, have a low volatility and are not harmful to health. Furthermore, the preparation of the plasticizers should be simple and, to meet ecological demands, should be carried out without producing waste materials such as by-products which cannot be recycled and wastewater containing pollutants.

Among the most important plasticizers are the esters of dicarboxylic and polycarboxylic acids with plasticizer alcohols, i.e. unbranched or branched primary alcohols having from about 6 to 12 carbon atoms, which can be used as individual compounds or as a mixture. The preparation of the esters has been carried out by the classical process by reacting the acids or acid anhydrides with an alcohol or a mixture of different alcohols in the presence of an acid, preferably sulfuric acid, as catalyst. The alcohol component is usually used in excess. Attempts have been made to counter adverse color and odor of the reaction product by targeted selection of the acid used as catalyst, by mild reaction conditions and by complicated purification measures.

A further development in the preparation of esters suitable as plasticizers constitutes the use metal-containing esterfication catalysts. Suitable catalysts are, for example, tin, titanium and zirconium which are used as finely divided metal or advantageously in the form of their salts, oxides or soluble organic compounds. These catalysts are high-temperature catalysts which reach their full activity only at esterification temperature above 180°C. Examples are tin powder, tin(II)oxide, titanate esters such as tetraisopropyl orthotitanate or tetrabutyl orthotitnate and also zirconium esters such as tetrabutyl zirconate. Alkyl titanates and titanium chelates, i.e. titanates of polyalcohol, have achieved particular importance in industrial production process.

Furthermore U.S. Patent 6,310,235 disclosed a process for preparing ester plasticizers by reacting dicarboxylic or polycarboxylic acids or their anhydride with alcohols in the presence of a titanium-containing, zirconium-containing or tin-containing catalyst. It comprises allowing a mixture of acid or acid anhydride and alcohol to react first at from 120 to 160°C, while removing any water formed, completing the reaction by addition of the catalyst and increasing the temperature to about 250°C, neutralizing the reaction mixture with an aqueous alkali metal hydroxide or alkaline earth metal hydroxide solution, then separating off the excess alcohol and drying and filtering the remaining crude ester. The process for preparing ester plasticizers is necessary for a high outlay in terms of time and energy.

U.S. Patent 6,284,917 and 6,888,021 disclosed a process for preparing ester plasticizers by hydrogenating benzenepolycarboxylic acid or derivatives selected from the group consisting of di-2-ethylhexyl phthalate and di-iso-nonyl phthalate thereof by using a catalyst containing macropores. This catalyst, containing macropores and applied to a support has a mean pore diameter of at least 50nm, is a kind of active metal catalyst which at least comprises one metal selected from transition group VIII of the Periodic Table alone or together with the other metal selected from transition group I or VII of the Periodic Table. The hydrogenation was carried out using pure hydrogen at 50 to 250°C and a constant pressure from 20 to 300 bar in the presence of ruthenium catalyst. The hydrogenation products obtained from the disclosed process for preparing ester plasticizers are applicably used as plasticizers in plastics, but the process for preparing ester plasticizers is necessary for a high outlay in terms of time and apparatus.

Besides, the highest amount of consumption of plasticizers such as di-2-ethylhexyl phthalate and di-iso-nonyl phthalate shall increasingly generate the problem of environmental hormone which seriously impaired environment sanitation and human body according to report.

### SUMMARY OF THE PRESENT INVENTION

It is therefore an object of the invention to improve the known process and, by optimizing and simplifying the process, to have the reaction product obtained from the invented process as more applicably employed in many applications as possible.

The invention provides a process for preparing ester plasticizers by reacting polycarboxylic acids or their anhydrides with alcohols in the presence of a titanium-containing, tin-containing or inorganic acid catalyst.

It comprises allowing a mixture of acid or acid anhydride and alcohol to react for single stage and 200 to 250°C at temperature, while removing any water formed, completing the reaction neutralizing the reaction mixture with an aqueous alkali metal hydroxide solution, then separating off the excess alcohol and filtering the remaining crude ester.

The invention can reduce the reaction time, and particularly the obvious advantage of the invention is that the reaction products obtained from the invented process have no any phathalic acid or anhydride.

The novel process of the invention has high reliability when implemented in industrial plants. It is easy to carry out, including continuously, and gives plasticizers of high purity and good color properties.

This reaction product has no any phathalate and has excellent processibility to prepare as a plasticizer containing cyclohexane polycarboxylic acid ester, it is also worth emphasizing that the plasticizer without any phathalate has no any harmful to the human's body and environment which makes it possible to use them widely for plastics in the field of children toy and food packaging and so on, so that it can supersede the phthalate group plasticizer.

In view of the above the purpose of this invention is to improve the producing method presently existing, to simplify the producing process, and optimizing the producing method, and to extend the field of application of the product obtained from the aforesaid reaction.

### DETAILED DESCRITION OF THE PREFERRED EMBODIMENTS

Acids suitable as staring materials for the process of the invention are polycarboxylic acids and derivatives.

Example of such compounds includes cyclohexane-1,2-dicarboxylic acid or its derivatives, cyclohexane-5-methyl-1,2dicarboxylic acid or its derivatives, cyclohexane-5-ethyl-1,2dicarboxylic acid or its derivatives, cyclohexane-1,3-dicarboxylic acid, cyclohexane-1,4-dicarboxylic acid, cyclohexane-1,2,3-tricarboxylic acid, or cyclohexane-1,2,4-tricarboxylic acid.

Alcohol used is, in particular, straight-chain or brached saturated aliphatic compounds having more than 4 carbon atoms in the molecule. They usually contain a primary hydroxyl group, but secondary alcohols are not ruled out as reaction partners of the acids. Example of such compounds includes butyl alcohol, n-amyl alcohol, hexyl alcohol, heptyl alcohol, n-octyl alcohol, n-nonyl alcohol, n-decyl alcohol, n-undecyl alcohol, n-dodecyl alcohol, iso-butaol, 2-butanol, tert-butyl alcohol, isoamyl alcohol, tert-amyl alcohol, 2-pentanol, 3-pentanol, iso-hexanol, 2-ethylhexyl alcohol, isononyl alcohol, isodecyl alcohol etc.

Esterification catalysts used in the novel process are metal catalysts and inorganic acid. The metal catalysts include titanium-containing catalyst and tin-containing catalyst which is formed as metals in finely divided form or preferably as compounds. Suitable compounds are tin(II)oxide, tin(II)oxalate, phenoxides, acylates and chelates of titanium and also esters of titanium e.g. tetraisopropyltitante, tetrabutyltitanate. The amount of catalyst used can extend over a wide range. Before esterification, it is possible to use larger amounts of catalyst from 0.4 to 6% by weight based on the reaction mixture. However, it is without addition of a catalyst during the end period of the reaction.

Although the esterification can be carried out using stoichiometric amounts of alcohol and acid, particularly when entrainers are used for removing the water of reaction, it is preferably to use a stoichiometric excess of the alcohol from 0.1 to 0.8 mole per mole of polycarboxylic acid or acid anhydride in order to achieve a conversion of the acid as completely as possible.

According to the invention, the esterification is carried out in single stage at high temperature, and characterized by treating the ester product obtained by reacting 1.0 mole cyclohexanepolycarboxylic acid or derivatives, with 2.2~3.8 mole an alcohol having 4-12 carbon atoms, in the presence of the above-described catalysts at temperature from 200 to 250°C. The reaction time accords to reaction condition, e.g. reactant or reaction temperature, usually from 5 to 8 hours. The reaction pressure is 5-760 mbar, the water formed is removed from the reaction system by an azeotrope with the alcohol. As long as the reaction temperature is above the boiling point of the azeotrope i.e. in a range from 90 to 180°C under atmospheric pressure.

The course and completion of the esterification can in this case be observed via the formation of water and acid number, while the acid number less than lmgKOH/g, the reaction is complete. The reaction mixture comprises not only the desired reaction product, viz the diester or polyester, but also, in particular, partially esterified dicarboxylic or polycarboxylic acids, excess alcohol and the catalyst.

To work up the crude ester plasticizer, the product from the reactor is first neutralized with alkali metal hydroxide. The alkaline reagent is here employed as an aqueous solution containing from 5 to 20% by weight, preferably from 9 to 16% by weight of the hydroxide, based on the solution. The amount of neutralizing agent to be used depends on the proportion of acid components, free acid and monoesters in the crude product. This proportion is determined in the form of the acid number (in accordance with ASTM-D1045). According to the invention, the alkaline reagent is added in an excess which corresponds to from 4 to 5 times of the amount which is stoichiometrically required to neutralize the H⁺ ions presented. The hydroxides are selected to use as aqueous solution having a particular concentration and in a defined excess, among which the sodium hydroxide is preferably selected, to ensure that the acidic constituents of the reaction mixture are precipitated in a crystalline, i.e. very readily filterable form. At the same time, the catalyst is largely decomposed to form likewise easily filterable products. The alkaline treatment of the crude ester is not tied to the maintenance of particular temperatures. It is advantageously carried out immediately after the esterfication step without prior cooling of the reaction mixture.

The excess of alkali metal hydroxide, regardless of only a small amount relative to the reaction product, reacts with the ester to form a carbonate salt which is obtained in crystalline form and can be filtered off without difficulty.

When the acid number of the reaction product is less than 0.08 mgKOH/g during the neutralization mentioned above, the free alcohol is separated from the reaction mixture. Steam distillation has been found to be useful for this step and can be carried out in simple form by passing steam into the crude product. An advantage of steam distillation is that last residues of catalyst are destroyed and converted into conveniently filterable hydrolysis products. For this purpose, it can be advantageous to add a high surface area adsorbent, e.g. activated carbon, to the reaction mixture prior to the distillation in order to aid the removal of the downstream products of the catalyst.

The removal of the free alcohol is followed by the drying of the ester. In a particularly simple and effective embodiment of this step, drying is achieved by passing an inert gas through the product. The crude ester is then filtered to free it of solid, viz the salts of (possibly partially esterified) carboxylic acids, hydrolysis products of the catalyst and the adsorbent. The filtration is carried out in conventional filtration equipment at room temperature or at temperatures up to room temperature. The filtration can use customary filter such as cellulose, kieselguhr or wood flour.

The resulting mixture is cyclohexanepolycarboxylic esters having purity of 99.8% or higher, good color of 10APHA and particularly without any phthalate, so that the cyclohexanepolycarboxylic esters can be applicably used for plasticizers.

The process of the invention dramatically reduces the reaction time and energy required for preparing the esters and is capably carried out economically on an industrial scale.

The plasticizers obtained from the invented process have the same excellent quality and processibility as the phthalate plasticizers e.g. DOP or DINP have. In particular, the plasticizers obtained from the invented process contain no any phathalate which are applicable in every respects required for high requirements of health and safety e.g. food packaging, film, toys, water beds, baby carriage, bottle caps, medical application, fabric coating, footwear, bloom bags, foil, sheeting and the like.

The process of the present invention is illustrated below by means of some examples. However, all of the examples presented here are only for illustration but not for restriction on the range of claims of the invention.

### EXAMPLES

The reaction mixture obtained by the esterification reaction of the following embodiment examples and comparison examples is measured its acid number (mgKOH/g) in accordance with ASTM-D1045; its purity by GC (Gas chromatography) and its color by Color of Pt-Co unit.

### Example 1

In a 1000ml four-neck reactor, 154g of hexahydrophthalic anhydride (HHPA), 370g of isononyl alcohol (INA), 2.6g of tetraisopropyl titanate (TIPT) catalyst were added. The esterification was carried out at 250°C and pressure of 5~760mbar for 5 hours with inert gas, water formed during the reaction is removed from the reaction system.

The completion of the esterification can be observed via acid number, when the acid number reduce 1.0mgKOH/g or less, subsequently neutralization with sodium hydroxide until the acid number reduce 0.08mgKOH/g or less. Subsequent to the neutralization the free INA is separated from the reaction mixture by steam distillation until the residual of INA reduce 300ppm or less. The removal of the free INA is followed by drying and filtration of the ester. The reaction resulting mixture of cyclohexane-1,2-dicarboxylic acid diisononyl ester was obtain by the process.

The analyst of the cyclohexane-1,2-dicarboxylic acid diisononyl ester is shown in table 1 having the color of 10APHA, the acid number of 0.05mgKOH/g and the purity of 99.8%. The resulting mixture can be used as plasticizer.

### Example 2

In a 1000ml four-neck reactor, 154g of hexahydrophthalic anhydride (HHPA), 370g of 2-ethylhexy alcohol (2-EH), 2.6g of tetraisopropyl titanate (TIPT) catalyst were added. The esterification was carried out at 200°C and pressure of 5~760mbar for 7 hours with inert gas, water formed during the reaction is removed from the reaction system.

The complection of the esterification can be observed via acid number, when the acid number reduce 1.0mgKOH/g or less, subsequently neutralization with sodium hydroxide until the acid number reduce 0.08mgKOH/g or less. Subsequent to the neutralization the free 2-EH is separated from the reaction mixture by steam distillation until the residual of 2-EH reduce 300ppm or less. The removal of the free 2-EH is followed by drying and filtration of the ester. The reaction resulting mixture of cyclohexane-1,2-dicarboxylic acid di(2-ethylhexyl) ester was obtain by the process.

The analyst is shown in table 1 having the color of 10APHA, the acid number of 0.07mgKOH/g and the purity of 99.6%. The resulting mixture can be used as plasticizer.

### Example 3

In a 1000ml four-neck reactor, 146.3g of hexahydrophthalic anhydride (HhIPA), 7.7g of 5-methyl-1,2-hexahydrophthalic anhydride (MHHPA), 370g of isononyl alcohol(INA), 2.6g of tetraisopropyl titanate (TIPT) catalyst were added. The esterification was carried out at 250°C and pressure of 5~760mbar for 8 hours with inert gas, water formed during the reaction is removed from the reaction system.

The complection of the esterification can be observed via acid number, when the acid number reduce 1.0mgKOH/g or less, subsequently neutralization with sodium hydroxide until the acid number reduce 0.08mgKOH/g or less. Subsequent to the neutralization the free INA is separated from the reaction mixture by steam distillation until the residual of INA reduce 300ppm or less. The removal of the free INA is followed by drying and filtration of the ester.

The reaction resulting mixture of cyclohexane-1,2-dicarboxylic acid diisononyl ester and 5-methyl-cyclohexane-1,2-dicarboxylic acid diisononyl ester was obtain by the process.

The analyst is shown in table 1 having the color of 10APHA, the acid number of 0.06mgKOH/g and the purity of 99.5%. The resulting mixture can be used as plasticizer.

### Comparison example 1

In a 1000ml four-neck reactor, 154g of hexahydrophthalic anhydride (HHPA), 370g of isononyl alcohol (INA), 2.6g of Para-tolune sulfonic acid (PTSA) catalyst were added. The esterification was carried out at 230°C and pressure of 5~760mbar for 8 hours with inert gas, water formed during the reaction is removed from the reaction system.

The complection of the esterification can be observed via acid number, when the acid number reduce 3.0mgKOH/g or less, subsequently neutralization with sodium hydroxide until the acid number reduce 0.08mgKOH/g or less. Subsequent to the neutralization the free INA is separated from the reaction mixture by steam distillation until the residual of INA reduce 300ppm or less. The removal of the free INA is followed by drying and filtration of the ester. The reaction resulting mixture of cyclohexane-1,2-dicarboxylic acid diisononyl ester was obtain by the process.

The analyst of the cyclohexane-1,2-dicarboxylic acid diisononyl ester is shown in table 1 having the color of 150 APHA, the acid number of 0.08mgKOH/g and the purity of 99.5%. The resulting mixture can be used as plasticizer.

### Comparison example 2

In a 1000ml four-neck reactor, 154g of hexahydrophthalic anhydride (HHPA), 370g of isononyl alcohol (INA), 2.6g of stannous octoate catalyst were added. The esterification was carried out at 230°C and pressure of 5~760mbar for 7 hours with inert gas, water formed during the reaction is removed from the reaction system.

The complection of the esterification can be observed via acid number, when the acid number reduce 1.0mgKOH/g or less, subsequently neutralization with sodium hydroxide until the acid number reduce 0.08mgKOH/g or less. Subsequent to the neutralization the free INA is separated from the reaction mixture by steam distillation until the residual of INA reduce 300ppm or less. The removal of the free INA is followed by drying and filtration of the ester. The reaction resulting mixture of cyclohexane-1,2-dicarboxylic acid diisononyl ester was obtain by the process.

The analyst of the cyclohexane-1,2-dicarboxylic acid diisononyl ester is shown in table 1 having the color of 40 APHA, the acid number of 0.07mgKOH/g and the purity of 99.6%. The resulting mixture can be used as plasticizer.

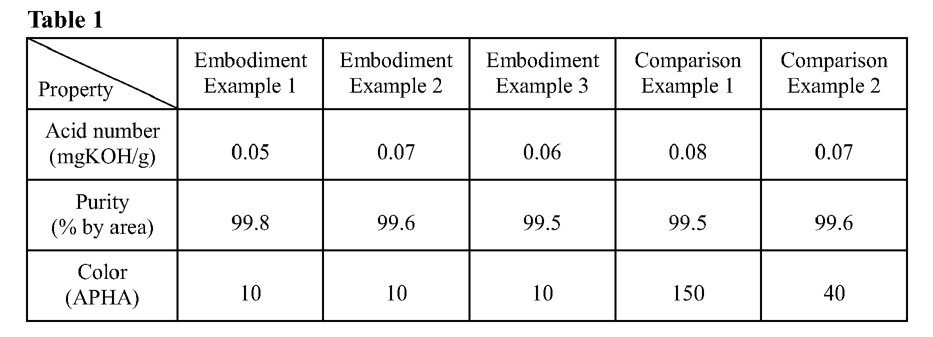

From the Table 1, it shows that the reaction product obtained by esterification in the presence of TIPT catalyst of the embodiment example 1 has the color, acid number and purity all better than that of comparison example 1 and comparison example 2; and the embodiment examples 2 and 3 has the color better than that of comparison example 1 and comparison example 2, too.

### Comparison example 3

The esterification was carried out in two stages. In the first stage, without addition of any catalyst, the monoester of the dicarboxylic acid is formed.

In a 1,000ml four-neck reactor, 154g of hexahydrophthalic anhydride, 370g of isononyl alcohol were added and carried out the reaction at temperature of 160°C. The course and completion of the esterification in this case can be observed via the formation of water.

In the second stage, the esterfication of the dicarboxylicis is then completed, it is carried out in the presence of 2.6g of TIPT catalyst at temperatures which are above those employed in the first stage and go up to about 250°C, and pressure of 5~760mbar for 11 hours, the water formed is removed out the reaction system.

The completion of the esterification can be observed via acid number, when the acid number reduce 1.0mgKOH/g or less, subsequently neutralization with sodium hydroxide until the acid number reduce 0.08mgKOH/g or less. Subsequent to the neutralization the free INA is separated from the reaction mixture by steam distillation until the residual of INA reduce 300ppm or less. The removal of the free INA is followed by drying and filtration of the ester. The reaction resulting mixture of cyclohexane-1,2-dicarboxylic acid diisononyl ester was obtain by the process.

The difference between Comparison Example 3 and Example 1 is illustrated in the Table 2.

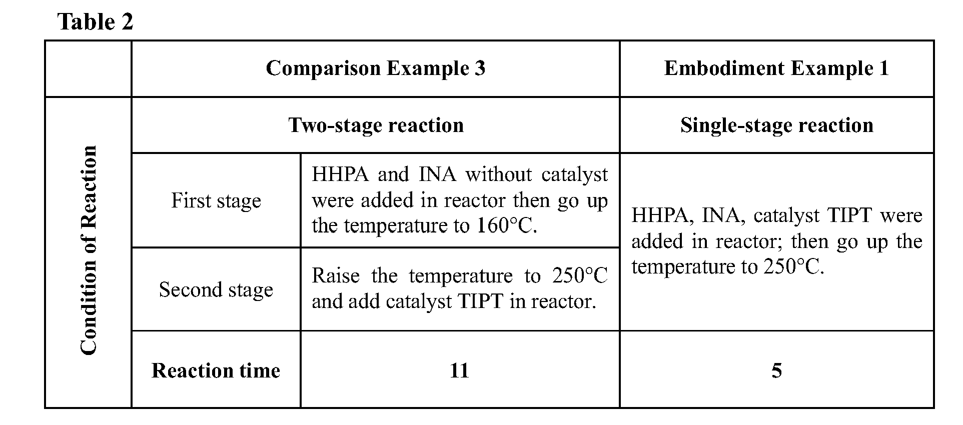

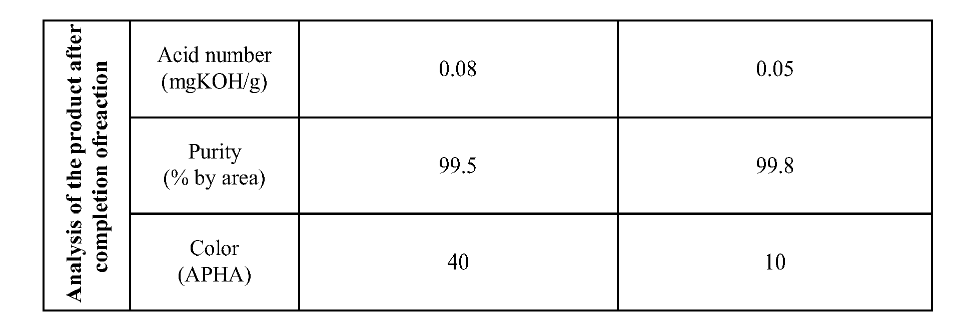

From the Table 2, it shows that the difference of the esterified products obtained from two different production processes can be clearly compared, and it can be show that the reaction product was obtained by the single-stage esterification of high temperature of the embodiment example 1 has the purity, acid number and color all better than that of the reaction product was obtained by the two-stage process of the comparison example 3.

Besides, the reaction time by single-stage process of the embodiment example 1 is 5 hours only much shorter than that of the comparison example 3 by 6 hours.

As for the comparison example 3 since the production process comprises two stage of reaction, and the catalyst was added in the second stage, the production process needs longer reaction time, and the quality of reaction product is poor as compared with that of the embodiment example 1 of the invention.

Therefore, the reaction product by single-stage at high temperature esterifiaction process of the invention can effectively shorten the production time, save production energy, reduce production cost, and obtain higher quality of the reaction product.

### Comparison example 4

Using the cyclohexane-1,2-dicarboxylic acid diisononyl ester was obtained by the above described process of the embodiment example 1 as plasticizer to compare the physical property of processability with the plasticizer of DOP and DINP.

The processability test is carried out by measuring 100g of PVC powder (DP=1000), 1.5g of calcium-zinc liquid stabilizing agent and 40g of cyclohexane-1,2-dicarboxylic acid diisononyl ester, DOP and DINP separately in each case. The test result is shown in Table 3.

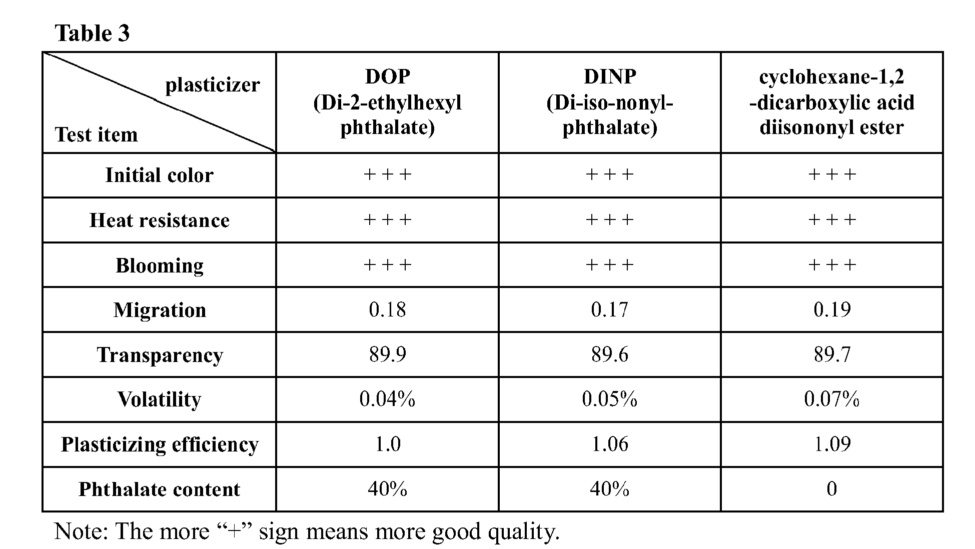

The test results shows that the plasticizer of cyclohexane polycarboxylic acid diisononyl ester was obtained by the described process of embodiment example 1 has the process ability equivalent to that of the DOP and DINP plasticizer in initial color, heat resistance, blooming, migration, transparency, volatility and plasticizing efficiency.

## Claims

1. A method for preparing cyclohexanepolycarboxylic ester plasticizer without phthalatic, **characterized in that** which comprises to have cyclohexane polycarboxylic acid or its derivatives reacted with mono-alcohol in the presence of a titanium-containing catalyst, tin-containing catalyst or inorganic acid at the same time added in a reactor to undergo a single-stage esterification reaction at temperature of 200 to 250°C and pressure of 5 to 760mbar for 5 to 8 hours to obtain a reaction mixture prepared for a plasticizer product without phthalatic; to neutralize the reaction mixture with an aqueous alkali metal hydroxide solution of 5~20% by weight based on the reaction mixture, under the condition of having the alkaline reagent added in an excess which corresponds to from 4 to 5 times of the amount stoichiometrically required to neutralize the H⁺ ions presented; and, subsequent to the neutralization, to separate the free alcohol from the reaction product and to remove the free alcohol followed by drying and filtration of the reaction product to obtain a plasticizer of cyclohexane polycarboxylic acid ester.

2. The method as described in claim 1, **characterized in that** the cyclohexane polycarboxylic acid and its derivatives include cyclohexane-1,2-dicarboxylic acid or its derivatives, cyclohexane-5-methyl-1,2dicarboxylic acid or its derivatives, cyclohexane-5-ethyl-1,2dicarboxylic acid or its derivatives, cyclohexane-1,3-dicarboxylic acid, cyclohexane-1,4-dicarboxylic acid, cyclohexane-1,2,3-tricarboxylic acid and cyclohexane-1,2,4-tricarboxylic acid; and the mono-alcohol includes unbranched or branched primary mono-alcohols having from about 4 to 12 carbon atoms.

3. The method as described in claim 1, **characterized in that** the amount of cyclohexane dicarboxylic acid or its derivatives employed 30 to 45 % by weight based on the total amount of the reactant.

4. The method as described in claim 1, wherein the amount of mono-alcohol employed 40 to 80 % by weight based on the total amount of the reactant.

5. The method as described in claim 1, **characterized in that** the catalyst used is inorganic acid including sulfuric acid, boric acid, phosphoric acid peroxochloric acid and p-toluenesulfonic acid.

6. The method as described in claim 1, **characterized in that** the catalyst contains metal and is selected from at lest one of the following groups of stannous octoate, tetra- isopropyl titanate (TIPT) and tetraisobutyltitanate (TIBP).

7. The method as described in claim 1, **characterized in that** the amount of catalyst employed 0.4 to 6 % by weight based on the total amount of reactant.

8. The method as described in claim 1, **characterized in that** the reactant is neutralized by aqueous solution of the hydroxide of alkaline metal, and the concentration of the aqueous solution is 9 to 16% by weight based on the solution.

9. The method as described in claim 1, **characterized in that** water of reaction formed during the reaction is removed from the reaction mixture by an azeotrope with the alcohol, and the catalyst is removed by steam and active carbon after completion of the esterification reaction.

10. A plasticizer obtained from the method as described in claim 1, **characterized in that** the composition of the plasticizer is cyclohexane polycarboxylic acid ester without any phathalate.
